(19) Europäisches Patentamt
European Patent Office
Office européen des brevets

(11) Veröffentlichungsnummer: **0 340 605**
**A2**

# (12) EUROPÄISCHE PATENTANMELDUNG

(21) Anmeldenummer: **89107439.5**

(22) Anmeldetag: **25.04.89**

(51) Int. Cl.4: **C07H 21/00 , C07H 23/00 , C12Q 1/68 , G01N 33/58**

(30) Priorität: **04.05.88 CH 1662/88**
**26.08.88 CH 3171/88**

(43) Veröffentlichungstag der Anmeldung:
**08.11.89 Patentblatt 89/45**

(84) Benannte Vertragsstaaten:
**BE CH DE ES FR GB IT LI NL SE**

(71) Anmelder: **F. HOFFMANN-LA ROCHE AG**
**Postfach 3255**
**CH-4002 Basel(CH)**

(72) Erfinder: **Bannwarth, Wilhelm, Dr.**
**Liebenbergstrasse 8**
**D-7888 Rheinfelden-Beuggen(DE)**
Erfinder: **Knorr, Reinhard, Dr.**
**Im Deichelacker 5**
**D-7859 Efringen-Kirchen(DE)**
Erfinder: **Müller, Francis**
**Seltisbergerstrasse 18**
**CH-4059 Basel(CH)**
Erfinder: **Schmidt, Dieter, Dr.**
**Redingstrasse 20**
**CH-4052 Basel(CH)**

(74) Vertreter: **Kloter, Rudolf et al**
**Postfach 3255 Grenzacherstrasse 124**
**CH-4002 Basel(CH)**

## (54) Rutheniumkomplex-Verbindungen.

(57) Es werden Rutheniumkomplex-Verbindungen, enthaltend eine DNA- oder RNA-Sequenz, die kovalent, vorzugsweise über eine Spacergruppe, an einen Rutheniumkomplex der allgemeinen Formel

$Ru^{2+}L_1L_2L_3$ I

gebunden ist, beschrieben. Die Reste $L_1$ und $L_2$ sind gleich oder verschieden und stellen Ladungsübertragungseinheiten dar. $L_3$ ist ebenfalls eine Ladungsübertragungseinheit, welche mit einer Gruppe A-X substituiert ist. Hierbei ist A eine Alkylengruppe, die gegebenenfalls mit $-SO_2-NH-$, -S-, -0-, -COO- oder mit -CO-NH- substituiert sein kann und X eine Aldehyd-, Carboxy-, Amino-, Hydroxy-, Thiocyanatgruppe, Halogen oder eine Phosphit- oder eine Phosphatgruppe oder eine modifizierte Phosphatgruppe, z.B. eine Phosphonat- oder Thiophosphonatgruppe oder eine sonstwie geeignete Funktion bedeutet. Die RNA- oder DNA-Sequenz ist entweder in modifizierter Form oder in unmodifizierter Form direkt oder über eine Spacer-Gruppe durch Reaktion mit dem Rutheniumkomplex kovalent verknüpft.

Die Ladungsübertragungseinheiten $L_1$, $L_2$ und $L_3$ können gleich oder verschieden sein und Bipyridyl, Bathophenanthrolin oder Banzbathophenantrolin darstellen, die gegebenenfalls substituiert sein können.

EP 0 340 605 A2

## Rutheniumkomplex-Verbindungen

Die vorliegende Erfindung betrifft Rutheniumkomplex-Verbindungen, enthaltend eine DNA- oder RNA-Sequenz, die kovalent, vorzugsweise über eine Spacer-Gruppe, an einen Rutheniumkomplex der allgemeinen Formel

$Ru^{2+}L_1L_2L_3$ I

gebunden ist, wobei $L_1$ und $L_2$ gleich oder verschieden sind und Ladungsübertragungseinheiten darstellen, und $L_3$ ebenfalls eine Ladungsübertragungseinheit darstellt, welche mit einer Gruppe A-X substituiert ist, wobei A eine Alkylengruppe, die gegebenenfalls mit $-SOH_2-NH-$, -S-, -O-, -COO- oder mit -CO-NH- substituiert sein kann und X eine Aldehyd-, Carboxy-, Hydroxy-, Amino-, Thiocyanatgruppe, Halogen oder eine Phosphit- oder Phosphatgruppe oder eine modifizierte Phosphatgruppe, z.B. eine Phosphonat- oder Thiophosphatgruppe, oder eine sonstwie geeignete Funktion darstellt, wobei die RNA- oder DNA-Sequenz entweder in modifizierter Form oder in unmodifizierter Form direkt oder über eine Spacer-Gruppe durch Reaktion mit dem Rutheniumkomplex kovalent verknüpft ist.

Die Ladungsübertragungseinheiten $L_1$, $L_2$ und $L_3$ können gleich oder verschieden sein und Bipyridil, Bathophenanthrolin oder Benzbathophenanthrolin darstellen, die gegebenenfalls substituiert sein können.

Bevorzugt ist eine Substitution der Ladungsübertragungseinheiten $L_1$ und $L_2$ mit Sulfonsäuregruppen.

Die Alkylengruppe A ist bevorzugt eine gerade oder verzweigte Alkylengruppe mit höchstens 8 C-Atomen.

Ganz besonders bevorzugt ist A eine $-(CH_2)_4-$ oder $-(CH_2)_5-$Gruppe.

Gemäss vorliegender Erfindung kann eine geeignete modifizierte DNA- oder RNA-Sequenz verwendet werden, wobei eine aminomodifizierte DNA- oder RNA-Sequenz besonders bevorzugt ist.

Im Prinzip kann jede gewünschte DNA oder RNA mit den Rutheniumkomplexen der Formel I gekoppelt werden, bevorzugt wird aber eine kovalente Kopplung der DNA oder RNA am 5′-Ende.

Die Rutheniumkomplexe der Formel I können gemäss Europäischer Patentanmeldung Nr. 85.1113777.9 (Veröffentlichungs-Nr. 178450) hergestellt werden oder in analoger Weise hierzu.

Die Kopplung der DNA- oder RNA-Sequenz erfolgt in an sich bekannter Weise. Eine mögliche Kopplungsweise mit modifizierter DNA oder RNA besteht darin, dass man den Rutheniumkomplex der Formel I und die modifizierte DNA- bzw. RNA-Sequenz mit einem wasserlöslichen Carbodiimidderivat, z.B. mit N-Cyclohexyl-N′-(2-morpholinoäthyl)-carbodiimidmethyl-p-toluolsulfonat behandelt. Ein besonders bevorzugtes Kopplungsmittel ist 1,1,3,3-Tetramethyl-2-succinimidyluronium Tetrafluoroborat (1:1) nachfolgend "TSU" genannt (das vorerwähnte "TSU" kann hergestellt werden, wie es in der offengelegten japanischen Patentanmeldung Nr. 166730/86 beschrieben ist). Die Kopplung erfolgt vorzugsweise in einem Lösungsmittelgemisch, z.B. aus DMF, Dioxan oder Wasser. Ueberraschenderweise wurde gefunden, dass eine Aktivierung von Carboxylfunktionen mit TSU auch in Anwesenheit von Wasser vonstatten geht.

Die Kopplung kann aber auch direkt erfolgen, z.B. über eine Phosphodiesterbindung, die in einem Lösungsmittel, wie Acetonitril oder absolutem Pyridin ausgebildet wird. Die Rutheniumkomplexe für eine direkte Kopplung werden in eine für die Kopplung geeignete Form überführt, vorzugsweise in Phosphoramidit- oder aktivierte Phosphatfunktionen.

Die Rutheniumkomplex-Verbindungen gemäss vorliegender Erfindung sind ausserordentlich geeignet zum Auffinden von komplementären DNA- oder RNA- Sequenzen. Dieses Auffinden kann nach der bekannten Hybridisierungtechnik erfolgen. Der Nachweis der Rutheniumkomplexe der Formel I, die als Marker-Moleküle dienen, erfolgt nach einer hochempfindlichen Fluoreszenz-Technik. Besonders bevorzugt ist die zeitaufgelöste Fluoreszenz-Technik, wie sie beispielsweise in der DTOS 2628158 beschrieben wird. Die Methodik dieser zeitaufgelösten Fluoreszenz-Technik ist im weitern in der schon früher erwähnten Europäischen Patentanmeldung Nr. 85.1113777.9 (Veröffentlichungs-Nr. 178450) beschrieben.

Im weiteren sind die Rutheniumkomplex-Verbindungen gemäss vorliegender Erfindung vorzugsweise geeignet zur Sequenzierung von Nukleotiden.

Ueberraschenderweise hat sich gezeigt, dass sich die hervorragenden Abklingzeiten der Rutheniumkomplexe der Formel I nicht wesentlich verändern, auch wenn diese an eine DNA-oder RNA-Sequenz gekoppelt sind, was sich sowohl in der Hybridisierungs-Technik wie in der Sequenzierung von Nukleotiden gezeigt hat. Des weiteren ändern sich die Abklingzeiten nicht wesentlich, wenn die Rutheniumkomplex-Verbindungen an einen Einzelstrang von DNA oder RNA hybridisieren.

Die Fluoreszenz der Rutheniumkomplex-Verbindungen ist $O_2$, pH und temperaturempfindlich.

Für Fluoreszenzmessungen hat sich, beispielsweise, die Verwendung von Tensiden und Antioxidanzien, auch mit dem Zusatz von Salzen wie $NH_4PF_6$ oder $NH_4BF_6$, als günstig erwiesen. Eine andere bevorzugte Möglichkeit besteht in der Verwendung von Thioglyzerin-Phthalsäure Lösungen. Es ist zu bemerken, dass

bei diesen bevorzugten Lösungen längere Abklingzeiten als in $H_2O$ gemessen werden können, ohne die Lösungen mit $N_2$ oder Ar zu begasen. Die Abklingzeit ist ein gut messbarer Parameter, der die Messbedingungen charakterisieren kann. In der folgenden Tabelle sind Abklingzeiten verschiedener Messlösungen als Beispiel aufgeführt.

| | | Abklingzeit $\tau$ in [$\mu$s] | | |
|---|---|---|---|---|
| Messlösung | Konzentration (% ww) | | +0.1M/l $Na_2SO_3$ | +0.1M/l $NH_4Cl_6$ +0.1M/l $Na_2SO_3$ |
| Thioglyzerin/Phthalsäure | | 7.2 | | |
| PBS, $H_2O$ | | 2.0 | 4.2 | |
| PBS + 12-DAPS | 0.7 | 1.3 | 1.9 | 7.5 |
| PBS + CHAPS | 2.5 | 3.0 | 1.9 | 5.2 |
| PBS + DDPC | 1.9 | 1.3 | 4.0 | 6.8 |
| PBS + $\beta$-OG | 3 | 1.9 | 1.7 | 4.7 |
| PBS + Triton X-100 | 2 | 1.9 | 2.2 | 5.4 |
| PBS + SDS | 2.9 | 1.2 | 6.0 | 7.4 |

PBS = Phosphate buffered saline (phosphatgepufferte Kochsalzlösung)
12-DAPS = Dimethylammoniopropansulfonat
CHAPS = 3-[(3-cholamidopropyl)-dimethyl-ammonio]-1-propansulfonat
DDPC = Dodecylphosphorycholin

-OG = Octyl- -D-glucopyranosid

SDS = Sodium-dodecyl-sulfate(Natriumdodecylsulfat)

Es versteht sich jedoch, dass nicht nur Messungen in Lösungen, sondern auch an festen Phasen vorgenommen werden können.

Die nachfolgenden Beispiele illustrieren die vorliegende Erfindung:

## Beispiele

5′-Amino-5′-desoxythymidin kann nach publizierten Wegen dargestellt werden, z.B. nach I. Yamamoto, M. Sekine, T. Hata; J. Chem. Soc. Perkin I, 1 (1980) 306 oder D.E. Gibbs, L.E. Orgel, J. Carbohydrates, Nucleosides, Nucleotides, 3, 5&6, (1976) 315.

## Beispiel 1

5′-Monomethoxytritylamino-5′-desoxythymidin. (1)

25 mmol (6,03 g) 5′-Amino-5′-desoxythymidin wurden zweimal nach Aufnehmen in abs. Pyridin engeengt. Dann wurde in 150 ml abs. Pyridin gelöst und mit 16.7 mmol (2,04 g) 4-Dimethylaminopyridin, 15,7 mmol (2,3 ml: 1,70 g) Triäthylamin sowie 60 mmol Monomethoxytritylchlorid versetzt und bei RT gerührt. Nach 2 h wurden 20 ml Methanol zugegeben und nach weiteren 15 Min. auf 250 ml gesättigte Natriumbicarbonatlösung gegossen und viermal mit je 250 ml Chloroform extrahiert. Die vereinigten organischen Phasen wurden über $Na_2SO_4$ getrocknet und nach Filtration vom Trockenmittel eingeengt.

Der Rückstand wurde über 200 g Kieselgel mittels Kurzsäulenchromatographie aufgetrennt. Die reinen Produktfraktionen wurden gesammelt und eingeengt. Der Rückstand wurde nach Lösen in 20 ml Chloroform und 3 ml Triäthylamin durch Eintropfen in 2 1 n-Pentan umgefällt. Das Präzipitat wurde gesammelt und

getrocknet und lieferte 6,2 g (48,3 %) reines Produkt.

Beispiel 2

5′-Monomethoxytritylamino-5′-desoxythymidin 3′-0- (2-cyanoäthyl N,N-diisopropylphosphoramidit) (2)

4 mmol (2,01 g) 5′-Monomethoxytritylamino-5′-desoxythymidin und 3 mmol Bis (diisopropylammonium) tetrazolid (0,55 g) wurden über Nacht am Hochvakuum getrocknet. Andertags wurde das 4′-Monomethoxyt ritylamino-5′-desoxythymidin in 60 ml wasserfreiem $CH_2Cl_2$ aufgenommen und mit dem Tetrazolid sowie 7,9 mmol (2,60 g) Bis-(diisopropylamino)-cyanoäthoxyphosphin versetzt. Nach 1 h wurde auf 70 ml gesättigte Bicarbonat-Lösung gegossen und mit $CH_2Cl_2$ extrahiert. Die vereinigten organischen Phasen noch zweimal mit je 50 ml gesättigter NaCl-Lösung ausgezogen und organische Phase eingeengt. Der Rückstand wurde in 20 ml $CH_2Cl_2$ gelöst und durch Eintropfen in 700 ml n-Pentan umgefällt. Nach Trocknen über Nacht wurden 2,8 g Produkt in Form eines farblosen Pulvers erhalten. (98 %)

Beispiel 3

Synthese von 5′-Amino-d[TAAAACGACGGCCAGTG (3a)

Der Aufbau der Sequenz bis zum letzten Baustein erfolgte an Controlled Pore Glas als festem Trägermaterial (6 mmol) nach bereits publizierter Methode (W. Bannwarth, P. Iaiza, DNA 5, 5 (1986) 413).

Nach Abspaltung der Dimethoxytritylschutzgruppe vom aufgebauten 16 mer wurde in einem üblichen Zyklus 5′- Monomethoxytritylamino-5′-desoxythymidin-3′-0-(2-cyanoäthyl N, N-diisopropylphosphoramidit) als Baustein für die letzte Kondensation eingesetzt. Nach Abspaltung der Schutzgruppen wurde das Rohgemisch mittels Polyacrylamidgelektrophorese sowie mit Umkehrphasen HPLC untersucht. Hierbei zeigte es sich, dass das gewünschte 5′-Amino- d[TAAAACGACGGCCAGTG] als Hauptprodukt im Rohgemisch enthalten ist (~ 60 %). Das Rohgemisch (490 OD, 260 nm) wurde zweimal dialysiert (gegen KCl) und in dieser Form in die Kopplungsreaktion mit den entsprechenden aktivierten Ru-Komplexen 4a-d eingesetzt.

Beispiel 4

Synthese von 5′-Amino d[TGACGTTGTAAAACGACGGCCAGTG] (3b)

Die Synthese wurde anlog derjenigen von 3a durchgeführt, nur dass diesmal von 1,22 µmol funktionalisiertem Trägermaterial ausgegangen wurde. Nach Schutzgruppenabspaltung und Dialyse gegen KCl wurden 71 OD (260 nm) rohes 3b erhalten, welches in dieser Form in die Kopplungsreaktion eingesetzt wurde.

Beispiel 5

Synthese von 5′-Amino-d[TTTTCTGGATCCCTGAGCCTGTTC] (3c)

Die Synthese erfolgte analog Beispiel 3, nur dass von 10 µmol funktionalisiertem Trägermaterial ausgegangen wurde. Nach Schutzgruppenabspaltung und Dialyse gegen KCl wurden 1700 OD (260 nm) rohes 5c erhalten, welches in dieser Form in die Kopplungsreaktion eingesetzt wurde.

## Beispiel 6

Synthese von 5′-Aminopropylmodifizierter DNA (3d)

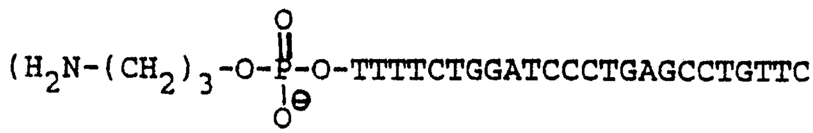

Der Aufbau der Sequenz d(TTTTCTGGATCCCTGAGCCTGTTC) erfolgte an Controlled Pore Gla als festem Trägermaterial (5 µmol) nach bereits publizierter Methode (W. Bannwarth, P. Iaiza, DNA 5,5 (1986) 413).

Nach Abspaltung der Dimethoxytrityl-Schutzgruppe wurde anschliessend in einem üblichen Zyklus (3-p-Methoxytritylaminopropoxy) (2-cyanoäthoxy)diisopropylaminophosphin als Bau stein eingesetzt (Dieser Baustein war aus β-Alanin-benzyl-ester in hoher Ausbeute in einer dreistufigen Synthese hergestellt worden).

Nach Abspaltung der Schutzgruppe wurde das Rohgemisch mittels Polyacrylamid-Gelelektrophorese untersucht, zweimal gegen Kaliumchlorid dialysiert und in dieser Form in die Kopplungsreaktion mit dem Komplex 5a eingesetzt.

## Beispiel 7

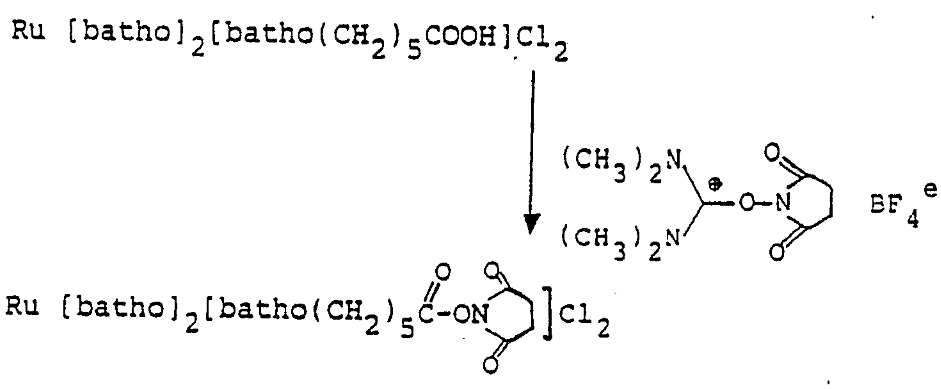

(4a)

(TSU)

(5a)

Im 10 ml Rundkolben mit $CaCl_2$-Röhrchen wurden 50 µmol (64,2 mg) Ru-Komplex 4a in 2 ml DMF (über Triphenylsilylchlorid destilliert) , aufgenommen. Bei RT wurden 55 µmol (18,0 mg) Aktivierungsagens (TSU) sowie 55 µmol (7,1 mg; 9,34 µl) Diisopropyläthylamin (Hünig-Base) zugegeben und 2 h gerührt. Das DMF wurde bei erhöhter Temperatur im Vakuum abgezogen und der Rückstand in Diäthyläther digeriert. Nach Filtration durch eine kleine Fritte wurde mehrmals mit Diäthyläther nachgewaschen und dann am Hochvakuum getrocknet, wobei 63 mg 5a als hellrotes Pulver erhalten wurden. (Rf = 0,56. Kieselgel HPTLC-Plättchen; in Diäthyläther vorlaufen lassen, dann in Chloroform/Methanol/Wasser (65/25/4; v/v). Obwohl 5a in dieser Form für die Kopplungsreaktionen eingesetzt werden kann, konnte man auch zeigen, dass sich 5a noch weiter durch Kurzsäulenchromatographie (B.J. Hunt, W. Rigby; Chem. Ind. (1967), 1868) auf Kieselgel mit $CHCl_3$ und steigendem Aethanolanteil reinigen lässt. Charakterisierung: $^1H$-NMR, IR.

## Beispiel 8

Ru $[batho]_2[batho(CH_2)_4COOH]Cl_2$ (4b)

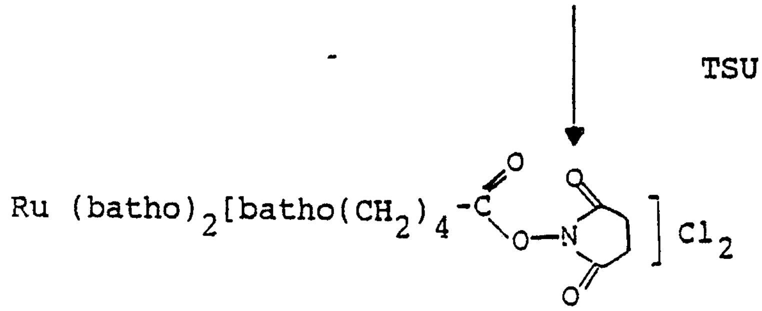


(5b)

Die Darstellung von 5b erfolgte in Analogie zu derjenigen von 5a, nur dass diesmal 100 µmol Hünig-Base eingesetzt und in 1,5 ml DMF während 6 h umgesetzt wurde. Die Aufarbeitung erfolgte analog Beispiel 6, wobei 105 mg 5b (76,9 %) erhalten wurden.

Eine kleine Menge wurde ebenfalls wieder mittels Kurzsäulenchromatographie über Kieselgel mit steigenden Aethanol Gradienten in $CHCl_3$ gereinigt. Identifizierung: $^1$H-NMR; IR.

## Beispiel 9

Ru $[batho]_2[benzbatho(CH_2)_4COOH]Cl_2$ (4c)

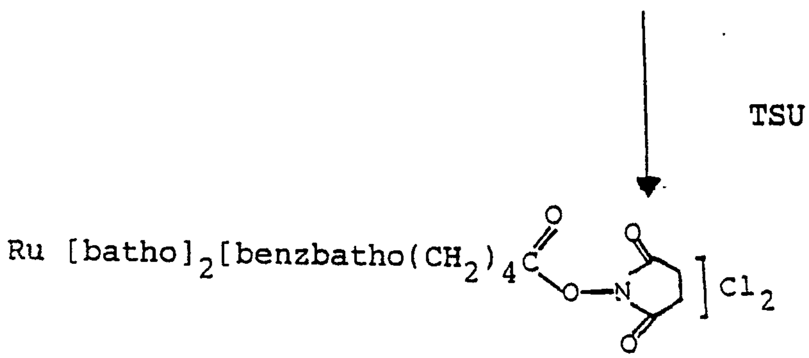


(5c)

Die Darstellung von 5c erfolgte analog Beispiel 8 ausgehend von den gleichen molaren Mengen 4c und den gleichen molearen Verhältnissen. Die Reaktionszeit betrug 2 h. Die Aufarbeitung erfolgte analog Beispiel 7. Ausbeute an 5c quantitativ. Ein kleiner Teil 5c wurde mittels Kurzsäulenchromatographie über Kieselgel mit steigendem Aethanol Gradienten in $CHCl_3$ als Laufmittel weiter aufgereinigt. 3 Analytik: $^1$H-NMR; IR

## Beispiel 10

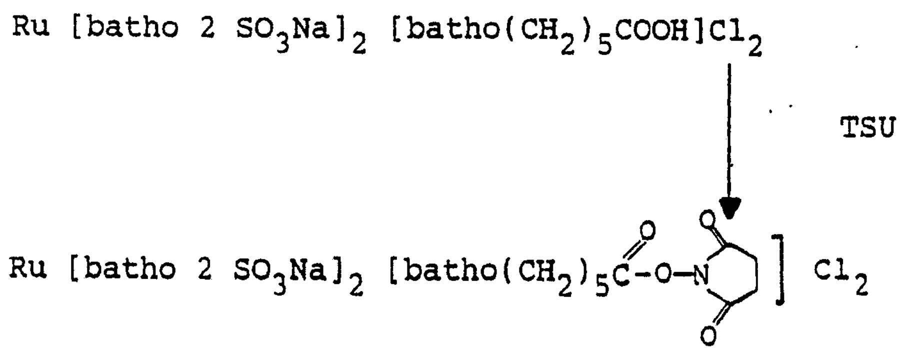


(4d)

(5d)

30 µmol (51 mg) 4d wurden mit 70 µmol (23,0 mg) TSU in Anwesenheit von 70 µmol (5,9 ml) Hünig Base in einem Gemisch aus 2 ml DMF und 0,2 ml $H_2O$ umgesetzt. Nach 1 h zeigte ein Dünnschichtchromatogramm vollständigen Umsatz. Das Lösungsmittel wurde am Rotationsverdampfer eingeengt und der Rückstand in Diäthyläther digeriert. Anschliessend wurde abgesaugt und mehrmals mit Diäthyläther nachgewaschen und getrocknet. Ausbeute an 5d quantitativ. Identifikation: IR.
Das Produkt wurde in dieser Form in die Kopplungsreaktionen eingesetzt.

## Beispiel 11

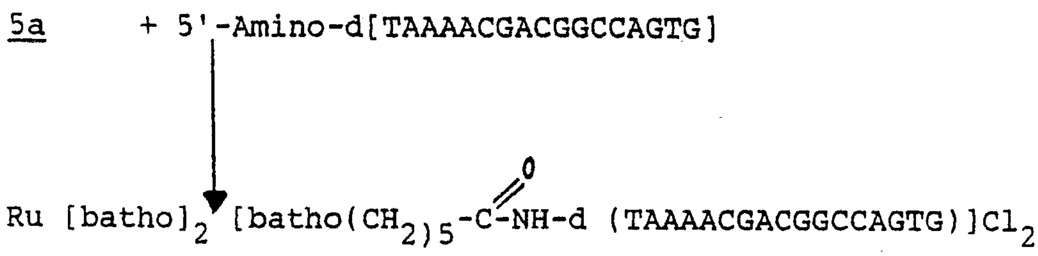

5a

(3a)

(6a)

40 OD Units (260 nm) an rohem, gegen KCl dialysiertem 5′-Amino-DNA Fragment 3a wurden in einem Gemisch aus 200 µl DMF, 200 µ1 Dioxan sowie 200 µl $H_2O$ aufgenommen; dazu wurden 6,0 mg (4,69 µmol 5a sowie 20 µmol (3,4 µl) Diisopropyläthylamin zugegeben und 24 h bei RT. im Dunkeln langsam geschüttelt. Das Lösungsmittel wurde am speed vac Concentrator abgezogen; der Rückstand in wenig Wasser aufgenommen und der überschüssige Komplex 5a mit $CHCL_3$ herausextrahiert. Die Aufreinigung der wässrigen Phase mittels HPLC auf reversed phase Kieselgelsäulen mit einem Laufmittelgradienten von 5-90 % Acetonitril in 0,1 M Triäthylammoniumacetat (pH 7,0) lieferte 6a in HPLC als auch Polyacrylamidgelelektrophoretisch reiner Form.

## Beispiel 12

5b + 3a → 6b

Die Darstellung erfolgte analog derjenigen von 5a (Beispiel 11), nur dass von 27 OD (260 nm) 3a sowie 2,34 µmol (3,2 mg) 5b (durch Kurzsäulenchromatographie gereinigt) und 10 µmol (1,7 µl) Hünig-Base in einem Gemisch aus 100 µl DMF, 100 µl Dioxan und 100 µl $H_2O$ umgesetzt wurde. Bei RT. wurde im Dunkeln für 142 h langsam geschüttelt. Die Isolierung von 6b erfolgte direkt durch HPLC Auftrennung eines Teils des Reaktionsgemisches auf C-18 Umkehrphasensäulen mit einem Gradienten von 5-90 % Acetonitril in 0,1 M Triathylammoniumacetat (ph 7,0). Das Produkt war gelchromatographisch einheitlich.

## Beispiel 13

5c + 3a → 6c

Die Umsetzung erfolgte analog Beispiel 12 mit den gleichen Reagenzien u. Lösungsmittelmengen. Auch die Aufarbeitung von 6c erfolgte analog, allerdings mit einem Gradienten von 30-90 % Acetonitril in 0,1 M Triäthylammoniumacetat. (pH 7). Das Produkt war gelchromatographisch einheitlich.

## Beispiel 14

5d + 3a → 6d

55 OD 3a wurden in einem Gemisch aus 200 µl DMF, 200 µl Dioxan und 200 µl $H_2O$ in Anwesenheit von 20 µmol (3,4 µl) Hünig Base mit 5 µmol (8,94 mg) 5d umgesetzt wie in den vorhergehenden Beispielen beschrieben. Nach 96 h Reaktionsdauer wurde 6d wie bereits beschrieben mittels HPLC isoliert. Das Produkt war gelchromatographisch einheitlich.

## Beispiel 15

5a + 3b → 6e

20 OD an 3b wurden in einem Gemisch aus 100 µl DMF, 100 µl Dioxan sowie 100 µl $H_2O$ in Anwesenheit von 10 µmol (1,7 µl) Hünig Base mit 2,32 µmol (3,2 mg) aktivierten Ru-Komplex 5a umgesetzt wie in den vorangegangenen Beispielen beschrieben. Nach einer Reaktionsdauer von 24 h wurde das Produkt 6e mittels Umkehrphasen HPLC isoliert.

## Beispiel 16

5a + 3c → 6f

40 OD an 3c wurden, wie bereits beschrieben, in einem Gemisch bestehend aus 200 µl DMF, 200 µl Dioxan sowie 200 µl $H_2O$ in Anwesenheit von 20 umol (3,4 µl) Hünig Base mit 6,4 mg (4,65 µmol) aktiviertem Ru-Komplex 5a unter Lichtanschluss für 24 h umgesetzt. Die Isolierung des Produktes 6f erfolgte wie beschrieben mit Umkehrphasen HPLC.

## Beispiel 17

Kopplung von 5a mit 3d

5a + 3d ⟶ 6g

10 OD'S an rohen, gegen Kaliumchlorid dialysierten 3d wurden mit 1,6 mg (1,17 µmol) 5a (mittels Kurzsäulen-Chromatographie gereinigt) in einem Gemisch bestehend aus 50 µl DMF, 50 µl Dioxan und 50 µl $H_2O$ in Anwesenheit von 0,85 µl Hünig-Base (5 µmol) im Dunkeln für 168 Std. umgesetzt. Das Rohgemisch wurde anschliessend am Speed Vac Konzentrator eingeengt. Der Rückstand wurde zweimal mit je 200 µl Methylenchlorid digeriert und jedesmal wurde der Rückstand nach dem Zentrifugieren entfernt. Der Rückstand wurde aufgenommen in 100 µl DMF und 100 µl Wasser, wobei eine vollständige Lösung entstand. Aus dieser wurde mittels HPLC auf C-18 Umkehrphasen-Säulen mit einem Gradienten von Acetonitril in 0,1 M Triäthylammoniumacetat (pH 7) das gewünschte Kopplungsprodukt in reiner Form erhalten.

## Beispiel 18

In situ Aktivierung und Kopplung von 5a mit 3a zu 6a 5µmol (6,4 mg) 5 wurden in einem Gemisch aus 200 µl DMF, 200 µl Dioxan sowie 100 µl $H_2O$ aufgenommen. Nach Zugabe von 10 µmol (3,3 mg) TSU und 20 µmol (3,4 µl) Hünig Base wurde 1 h leicht geschüttelt und das Reaktionsgemisch zu 32 OD rohem dialysiertem 3a gegeben und unter leichtem Schütteln unter Lichtausschluss 22 h reagieren lassen. Die weitere Aufreinigung und Isolierung erfolgte analog Beispiel 10.

## Beispiel 19

Anknüpfung des Ru-Komplexes über Phosphodiesterbindung

a) Ausgehend von 200 mg Trägermaterial (7,3 µmol) wurde die folgende Sequenz d-($^{5'}$GTTGACAAGAATCCTCACAATACC$_{3'}$) in üblicher Weise synthetisiert und davon 5 mg Trägermaterial abgezweigt. Nach Abspaltung der DMT-Schutzgruppe am 5'-Ende mit Dichloressigsäure (3%) wurde mit Acetonitril absolutiert. Parallel dazu wurde in situ das Phosphoramidit des Ru-Komplexes wie folgt hergestellt: 25 mg (20 µmol) Ru [batho]$_2$ [batho$(CH_2)_5$ OH] $Cl_2$ werden dreimal mit trockenem Acetonitril eingeengt und dann in 0,2 ml Acetonitril aufgenommen. Diese Lösung wurde versetzt mit 5,1 mg (30 µmol) Diisopropylammoniumtetrazolid sowie mit 0,1 ml einer Lösung, die 6 mg (20 µmol) Bis-(diisopropylamino)-ß-cyano-äthoxy-phosphin enthielt. Nach einer Reaktionszeit von 3 h wurde dieses Gemisch zum trockenen Trägermaterial hinzugegeben, welches die obige Sequenz bis auf die 5'-Hydroxylfunktion in geschützter Form trägt. Nach der Zugabe von 0,7 ml 0,48 M Tetrazollösung in Acetonitril wurde während 10 Min. reagieren lassen. Anschliessend wurde das Reaktionsgemisch vom Trägermaterial entfernt und zur Oxidation mit einer 0,2 M Jodlösung in einem Gemisch THF/Lutidin/ Wasser (800/200/20; v/v) reagieren lassen. Nach Entfernen dieser Lösung wurde mit Acetonitril sowie mit Aether gewaschen und das Trägermaterial getrocknet.

5 mg dieses Materials wurden für die Abspaltung der Schutzgruppen sowie das Loslösen vom Trägermaterial in 500 ml conc. Ammoniak aufgenommen und im gut verschlossenen Eppendorf Röhrchen 90 Min. bei 66°C gehalten. Nach Abkühlen wird die Lösung vom Trägermaterial abgetrennt und eingeengt. Das Pellet wurde aufgenommen in 100 ml Wasser und 200 ml Dioxan, digeriert und nach Zentrifugieren und Abheben wird mit 600 ml THF gefällt. Nach Zentrifugieren wurde das Pellet in Wasser aufgenommen und die gewünschte Verbindung nach Auftrennung mittels Umkehrsäulenchromatographie isoliert. Alternativ dazu lässt sich die Verbindung auch über Polyacrylamidgelelektrophoresen mit nachfolgender Elektroelution gewinnen.

b) Analog Beispiel a) wurde die DNA Sequenz auf dem Trägermaterial synthetisiert und für eine Ankopplung des Ru-Komplexes vorbereitet. Parallel dazu wurden 25 mg (20 µmol) des Ru-Komplexes wie in a) beschrieben in das entsprechende Phosphoramidit überführt. Nach einer Reaktionsdauer von 3 h wurde auf 50 ml ges.

$NaHCO_3$-Lösung gegossen. Es wurde dreimal mit $CH_2Cl_2$ extrahiert, die vereinigten organischen Phasen über $Na_2SO_4$ getrocknet und eingeengt. Das Einengen wurde noch dreimal nach Zugabe von abs.

Acetonitril wiederholt und der Rückstand dann in 0,3 ml abs. Acetonitril aufgenommen. Diese Lösung wurde dann wie bei a) zum Trägermaterial gegeben, ebenso wie 0,7 ml 0,48 M (333 µmol) Tetrazollösung in abs. Acetonitril. Nach einer Reaktionsdauer von 10 Min. wurde wie in a) beschrieben weitergearbeitet.

## Beispiel 20

## Sequenzierung nach der Didesoxy-Methode

Für die Ermittlung einer DNA-Sequenz nach der von Sanger (F. Sanger et al. PNAS 74 (1980) pp 6463ff) entwickelten Didesoxy- oder Kettenabbruchmethode ist die spezifische Hybridisierung des Sequenzierungsprimers an das zu sequenzierende Template eine der Grundvoraussetzungen. Es konnte gezeigt werden, dass ein mit einem Ru-Komplex markierter Sequenzierungsprimer zum gleichen Sequenzierungsmuster führt wie der entsprechende nichtmarkierte Primer. Damit wurde bewiesen, dass der Ru-Komplex-markierte Primer spezifisch hybridisiert und gleichzeitig wurde seine Eignung für Sequenzierung nachgewiesen. Die Sequenzierung wurde durchgeführt mit einem Sequenzierungskit der United States Biochemical Corp. (Art. No. 70700) und nach der im Kit angegebenen Methode. Als Sequenzierungsprimer wurden eingesetzt:

1) d(GACGTTGTAAAACGACGGCCAGTG)

2) 5′Amino d(TGACGTTGTAAAACGACGGCCAGTG) (3b)

3) 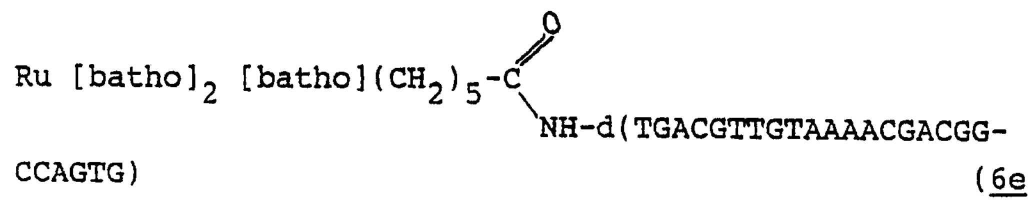

(6e)

= 3b

Die radioaktive Markierung der Sequenzierungansätze geschah durch Einbau von α-$^{32}$P-dATP. Nach Auftrennung der mit den obigen Primern parallel durchgeführten Sequenzierreaktionen mittels Gelelektrophorese und Autoradiographie ergibt sich für alle 3 Sequenzierungsreaktionen ein gleiches Sequenzierungsmuster.

Im einzelnen wurde diese Sequenzierungsreaktion wie folgt durchgeführt:

Zunächst wurden die folgenden Mischungen hergestellt.

### 1. Hybridisierungs-Gemische

1 µl (0,2 µg) Einzelstrang-Template M13 mp18 in 10 mM Tris/HCL pH 7,5; 1 mM EDTA
5 µl Wasser
2 µl 5x Sequenzierungs-Puffer:
0,2 M Tris/HCL pH 7,5
0,1 M Magnesiumchlorid
0,25 M Natriumchlorid
2 µl (6 x $10^{-5}$ OD) der jeweiligen Primer Lösung.
Das Gesamtvolumen war 10 µl.

Die Hybridisierungs-Gemische wurden jeweils 2 Min. auf 65 °C erhitzt und dann während 30 Min. auf Raumtemperatur abgekühlt. Während dieser Zeit wurden die folgenden Lösungen hergestellt:

### a) Terminations-Gemische

Für jede Sequenzierung mit einem der angegebenen Primer wurden vier Eppendorf-Röhrchen mit A, T, G und C beschriftet und anschliessend wird jedes Röhren mit 2,5 µl des entsprechenden Terminations-Gemisches versetzt, auf Eis aufbewahrt und kurz vor Gebrauch auf 37° C erwärmt.

| | |
|---|---|
| dd<br>A-Terminations-Gemisch: | 80 µM dGTP; 80 µM dATP; 80 µM dCTP;<br>80 µM dTTP; 8 µM ddATP; 50 mM NaCl |
| dd<br>C-Terminations-Gemisch: | 80 µM dGTP; 80 µM dATP; 80 µM dCTP;<br>80 µM dTTP; 8 µM ddCTP; 50 mM NaCl |
| dd<br>G-Terminations-Gemisch: | 80 µM dGTP; 80 µM dATP; 80 µM dCTP;<br>80 µM dTTP; 8 µM ddGTP; 50 mM NaCl |
| dd<br>T-Terminations-Gemisch: | 80 µM dGTP; 80 µM dATP; 80 µM dCTP;<br>80 µM dTTP; 8 µM ddTTP; 50 mM NaCl |

b) Markierungs-Gemisch

1,5 µM dGTP; 1,5 µM CTP; 1,5 µM TTP.

c) Enzym-Lösung

1 µl Sequenase ™ (Modifizierte DNA-Polymerase) einer Stammlösung, die 12,5 U/µl enthält, wurde mit 7 µl Wasser verdünnt.

2. Markierungs-Reaktionen

Zu jedem Hybridisierungsgemisch wurden die folgenden Lösungen hinzugegeben.
(10 µl Hybridisierungsgemisch)
1 µl 0,1 M DTT
2 µl Markierungs-Gemisch
1,5 µl α-$^{32}$P-dATP; 10 mCi/ml; 6000 Ci/mmol (Amersham International; Code: PB 10474)
2 µl Enzym-Lösung (3,1 U)
Gesamtvolumen ist 16,5 µl.
Die Lösungen wurden nach Durchmischen 5 Min. bei Raumtemperatur inkubiert.

3. Kettenabbruch-Reaktionen

Zu jedem Röhrchen mit den 4 verschiedenen Kettenabbruchsgemischen wurden 3,5 µl Markierungsgemisch zugegeben und 5 Min. bei 37° C inkubiert. Die Reaktionen mit den Primern 1 und 2 wurden gestoppt durch die Zugabe von 4 µl Farbstofflösung und zweiminütiges Erhitzen auf 95° C und Abschrecken im Eisbad. Sie sind dann bereit zum Aufladen auf das Polyacrylamidgel. Die Reaktion mit Primer 3 wurde ohne Zugabe von Farbstofflösung 2 Min. auf 95° C erhitzt und auf Eis abgeschreckt. Dann wurde mit 43 µl Wasser, 1 µl Glykogenlösung (20 mg/ml) sowie 100 µl Isopropanol versetzt. Nach 30 Min. bei -80° C wurde 15 Min. bei 0° C mit 12'000 RPM zentrifugiert. Der Ueberstand wurde entfernt und das Pellet in 5 µl Farbstofflösung aufgenommen. Nach Erhitzen auf 95° C (2 Min.) und Abschrecken auf Eis wurde auf Polyacrylamidgel aufgetragen.

4. Polyacrylamidgel

Ein 6% denaturierendes Polyacrylamidgel (20 x 40 x 0,04 cm) wurde 1,5 Std. preelektrophoretisiert (2'000 Volt; 13 mA) und dann wurden pro Slot 2 µl der jeweiligen Sequenzierungsreaktionen aufgetragen. Elektrophorese: 90 Min.; 2'500 Volt/14 mA). Anschliessend wurde ein Röntgenfilm exponiert, wobei bei allen 3 Primern das gleiche spezifische Sequenzierungsmuster erhalten wurde (vgl. hierzu Figur 1).

Beispiel 21

Spezifische Hybridisierung sowie deren Nachweis mittels Southern Hybridisierung

Für die Ermittlung der Spezifität Ru-Komplex markierter DNA-Fragmente wurde M13mp18 mittels des Restriktionsenzyms Ssp I verdaut und ansschliessend werden die gebildeten Fragmente gelelektrophoretisch aufgetrennt. Anschliessend erfolgte eine Uebertragung der Fragmente auf eine für Hybridisierungen geeignete Membran. Ein synthisches Oligonukleotid bestehend aus 17 Basen, welches komplementär zu einem Teil des Fragmentes mit 1554 bp ist, wurde in der beschriebener Art mit dem Ru-Komplex markiert (Beispiel 13). Ein kleiner Teil diese Ru-Komplex markierten Oligonukleotids wurde noch zusätzlich an 3'-Ende radioaktiv markiert. Anschliessend konnte gezeigt werden, dass sowohl die Probe, die sowohl mit Ru-Komplex als auch radioaktiv markiert war, als auch die nur mit dem Ru-Komplex markierte Probe mit dem entsprechenden Restriktionsfragment (1554 bp) spezifisch hybridisieren. Dies geschah zum einen durch Lokalisierung nach Auflegen eines Röntgenfilms als auch nach Ablösen der Probe und Messen der Radioaktivität und auch durch Messen der zeitaufgelösten Fluoreszenz der nach der spezifischen Hybridisierung abgelösten Ru-Komplex markierten Probe.

Im einzelnen wurde die Hybridisierung wie folgt vorgenommen:

Mit Hilfe von 30 Einheiten des Restriktionsenzyms Ssp I wurden 5 µg M13 mp 18 Rf-DNA in einem Gemisch bestehend aus 33 mM Tris/acetat (pH 7,9), 66 mM KOAc, 10 mM Mg $(OAc)_2$ und 0,5 mM DDT während 2 Std. bei 37° C gespalten. Die aus der Verdauung resultierenden Fragmente wurden anschliessend elektrophoretisch mittels eines 0,7% Agarose-Gels aufgetrennt, welches noch 300 ng/ml Ethidiumbromid enthielt. Nachdem das 1554 bp-Fragment etwa 8,5 cm gewandert war (Figur 2a) wurde das Gel für 15 Min. in 0,5 M NaOH, dann in 1 M NaOH und schliesslich noch zweimal in 0,5 M Tris/HCL pH 8,9; 3 M NaCl eingeweicht. Anschliessend erfolgte in 20 x SSC (3 M NaCl; 0,28 M Na-Citrat) über Nacht der Transfer auf eine Cellulosemembran. Die Membran wurde getrocknet und in 1 cm breite Streifen geschnitten, entsprechend den Bahnen der Ssp I Verdauung von M13 mp 18. Zwei solcher Streifen (entsprechend 1 µg Verdauung) wurden nach einer Vorhybridisierung mit Kalbs-Thymus DNA sowohl mit der Probe 6d als auch mit6d, welches noch mit Hilfe von $\alpha$-$^{32}P$ ddATP und terminater Nukleotidyl-Transferase radioaktiv markiert worden war, für 3 Std. bei 37° C hybridisiert.

Nichtgebundene DNA wurde mit Hilfe von 2 x SSC heruntergewaschen. Ein anschliessendes Autoradiogramm zeigte, dass die radioaktiv markierte Probe 6d spezifisch an das 1554 bp Fragment hybridisiert hatte, welches die zu 6d komplementäre DNA-Sequenz enthielt (Figur 2b). Die Hybridisierungsstreifen mit 6d wurden in Quadrate aufgeteilt und jedes Quadrat wurde wurde einzeln dreimal bei 65° C für 10 - 20 Min. mit 300 µl 0,1 x 55 C gewaschen. Die Waschflüssigkeiten wurden separat eingeengt und dann jeweils in 75 µl Wasser aufgenommen, worauf in Quadrat 9 Fluoreszenz nachgewiesen wurde.

## Ansprüche

1. Rutheniumkomplex-Verbindungen enthaltend eine DNA-oder RNA-Sequenz, die kovalent, vorzugsweise über eine Spacer-Gruppe, an einen Rutheniumkomplex der allgemeinen Formel

$Ru^{2+}L_1L_2L_3$ I

gebunden ist, wobei $L_1$ und $L_2$ gleich oder verschieden sind und Ladungsübertragungseinheiten darstellen, und $L_3$ ebenfalls eine Ladungsübertragungseinheit darstellt, welche mit einer Gruppe A-X substituiert ist, wobei A eine Alkylengruppe, die gegebenenfalls mit $-SO_2-NH-$, -S-, -O-, -COO-oder mit -CO-NH- substituiert sein kann und X eine Aldehyd-, Carboxy-, Amino-, Hydroxy-, Thiocyanatgruppe, Halogen oder eine Phosphit- oder eine modifizierte Phosphatgruppe, z.B. eine Phosphonat- oder Thiophosphatgruppe oder eine sonstwie geeignete Funktion darstellt, wobei die RNA- oder DNA-Sequenz entweder in modifizierter Form oder unmodifizierter Form direkt oder über eine Spacer-Gruppe durch Reaktion mit dem Rutheniumkomplex kovalent verknüpft ist.

2. Rutheniumkomplex-Verbindungen nach Anspruch 1, dadurch gekennzeichnet, dass die Ladungsübertragungseinheiten $L_1$, $L_2$ und $L_3$ gleich oder verschieden sind und Bipyridyl, Bathophenanthrolin oder Benzbathophenanthrolin-Gruppen darstellen, die gegebenenfalls substituiert sein können.

3. Rutheniumkomplex-Verbindungen nach Anspruch 1 oder 2, dadurch gekennzeichnet, dass die Ladungsübertragungseinheiten $L_1$ und $L_2$ mit Sulfonsäuregruppen substituiert sind.

4. Rutheniumkomplex-Verbindungen nach einem der Ansprüche 1-3, dadurch gekennzeichnet, dass A eine gerade oder verzweigte Alkylengruppe mit höchstens 8 C-Atomen darstellt.

5. Rutheniumkomplex-Verbindungen nach Anspruch 4, dadurch gekennzeichnet, dass A $-(CH_2)_4-$ bedeutet und X COOH bedeutet.

6. Rutheniumkomplex-Verbindungen nach Anspruch 4, dadurch gekennzeichnet, dass A die Gruppe $-(CH_2)_5-$ ist und X die Gruppe COOH oder eine Phosphoramidit- oder eine aktivierte Phosphatgruppe bedeutet.

7. Rutheniumkomplex-Verbindungen nach einem der Ansprüche 1-6, dadurch gekennzeichnet, dass eine nicht modifizierte oder eine aminomodifizierte DNA- oder RNA-Sequenz an den Rutheniumkomplex der Formel I gebunden ist.

8. Rutheniumkomplex-Verbindungen nach einem der Ansprüche 1-7, dadurch gekennzeichnet, dass die DNA- oder RNA-Sequenz am 5′-Ende an den Rutheniumkomplex der Formel I gekoppelt ist.

9. Rutheniumkomplex-Verbindungen nach einem der Ansprüche 1-8, dadurch gekennzeichnet, dass die DNA- oder RNA-Sequenz mittels eines Kopplungsmittels kovalent an den Rutheniumkomplex der Formel I gebunden ist.

10. Rutheniumkomplex-Verbindungen nach Anspruch 9, dadurch gekennzeichnet, dass das Kopplungsmittel ein Carbodiimidderivat ist.

11. Rutheniumkomplex-Verbindungen gemäss Anspruch 10, dadurch gekennzeichnet, dass das Carbodiimidderivat N-Cyclohexyl-N-( 2-Morpholinaethyl)-Carbodiimid-methyl-p-toluolsulfonat bedeutet.

12. Rutheniumkomplex-Verbindungen gemäss Ansprüch 9, dadurch gekennzeichnet, dass das Kopplungsmittel ein 1,1,3,3-Tetramethyl-2-succinimidyluronium Tetrafluoroborat (1:1) ist.

13. Rutheniumkomplex-Verbindungen gemäss Anspruch 8, dadurch gekennzeichnet, dass die DNA- oder RNA-Sequenz in unmodifizierter Form an den Rutheniumkomplex der Formel I gebunden ist.

14. Rutheniumkomplex-Verbindungen gemäss Anspruch 13, dadurch gekennzeichnet, dass die DNA- oder RNA-Sequenz über eine Phosphodiester-Gruppe an den Rutheniumkomplex der Formel I gebunden ist.

15. Rutheniumkomplex-Verbindungen nach Anspruch 1, dadurch gekennzeichnet, dass $L_1$ und $L_2$ Bathophenanthrolin-Reste bedeuten und $L_3$ eine Bathophenanthrolin-Gruppe bedeutet, A eine $-(CH_2)_5-$Gruppe bedeutet und X COOH bedeutet, und die 5′-Amino-DNA-Sequenz TAAAACGACGGCCAGTG$_3$′ ist.

16. Rutheniumkomplex-Verbindungen nach Anspruch 1, dadurch gekennzeichnet, dass $L_1$ und $L_2$ Bathophenanthrolin-Gruppen bedeuten und $L_3$ eine Bathophenanthrolin-Gruppe bedeutet, A die Gruppe $-(CH_2)4$ bedeutet und X eine COOH-Gruppe ist und die 5′-Amino-DNA-Sequenz TAAAACGACGGCCAGTG$_3$′ ist.

17. Rutheniumkomplex-Verbindungen nach Anspruch 1, dadurch gekennzeichnet, dass $L_1$ und $L_2$ Bathophenanthrolin-Gruppen bedeuten und $L_3$ eine Benzbathophenanthrolin-Gruppe bedeutet, A die Gruppe $-(CH_2)_4$ bedeutet und X eine COOH-Gruppe ist und die 5′-Amino-DNA-Sequenz TAAAACGACGGCCAGTG$_3$′ ist.

18. Rutheniumkomplex-Verbindungen nach Anspruch 1, dadurch gekennzeichnet, dass $L_1$ und $L_2$ durch Sulfonsäuregruppen substituierte Bathophenanthrolin-Gruppen bedeuten und $L_3$ eine Bathophenanthrolin-Gruppe bedeutet, A eine Gruppe $-(CH_2)5$ bedeutet und X eine -COOH-Gruppe bedeutet und dass die 5′-Amino-DNA-Sequenz TAAAACGACGGCCAGTG$_3$′ ist.

19. Rutheniumkomplex-Verbindungen nach Anspruch 1, dadurch gekennzeichnet, dass $L_1$ und $L_2$ durch Sulfonsäuregruppen substituierte Bathophenanthrolin-Gruppen bedeuten und $L_3$ eine Bathophenanthrolin-Gruppe bedeutet, A die Gruppe $-(CH_2)_5$ bedeutet und X eine -COOH-Gruppe bedeutet und dass die 5′-Amino-DNA-Sequenz TTTTCTGGATCCCTGAGCCTGTTC$_3$′ ist.

20. Rutheniumkomplex-Verbindungen nach Anspruch 1, dadurch gekennzeichnet, dass $L_1$ und $L_2$ Bathophenanthrolin-Gruppen bedeuten und $L_3$ eine Bathophenanthrolin-Gruppe bedeutet, A eine Gruppe $-(CH_2)_5$-Gruppe bedeutet und X eine -COOH-Gruppe bedeutet und dass die 5′Amino-DNA-Sequenz TGACGTTGTAAAACGACGGCCAGTG$_3$′ ist.

21. Rutheniumkomplex-Verbindung nach Anspruch 1, dadurch gekennzeichnet, dass $L_1$ und $L_2$ Bathophenthrolingruppen bedeuten und $L_3$ eine Bathophenanthrolingruppe bedeutet, A die Gruppe $-(CH_2)_5-$ bedeutet, X eine Phosphodiesterfunktion bedeutet und die DNA-Sequenz $^5$ GTTGACAAGAATCCTCACAATACC$_3$′ ist.

22. Verwendung einer Rutheniumkomplex-Verbindung nach einem der Ansprüche 1-21 zum Nachweisen von DNA- oder RNA-Sequenzen.

23. Verwendung nach Anspruch 22, dadurch gekennzeichnet, dass zum Nachweis die Hybridisierungstechnik verwendet wird.

24. Verwendung nach einem der Ansprüche 22 oder 23, dadurch gekennzeichnet, dass man die zeitaufgelöste Fluoreszenz-Technik anwendet.

25. Verwendung einer Rutheniumkomplex-Verbindung nach einem der Ansprüche 1-21 zum Sequenzieren von Nukleotiden.

26. Verwendung nach Anspruch 25, dadurch gekennzeichnet, dass man die zeitaufgelöste Fluoreszenz-Technik anwendet.

Figur 1

Bahn 1:

d(GACGTTGTAAAACGACGG-CCAGTG) als Primer

Bahn 2:

3b als Primer (nur beim starken Exponieren sichtbar)

Bahn 3:

6e als Primer

G, A, T, C:

Jeweils spezifische Reaktion für die Base.

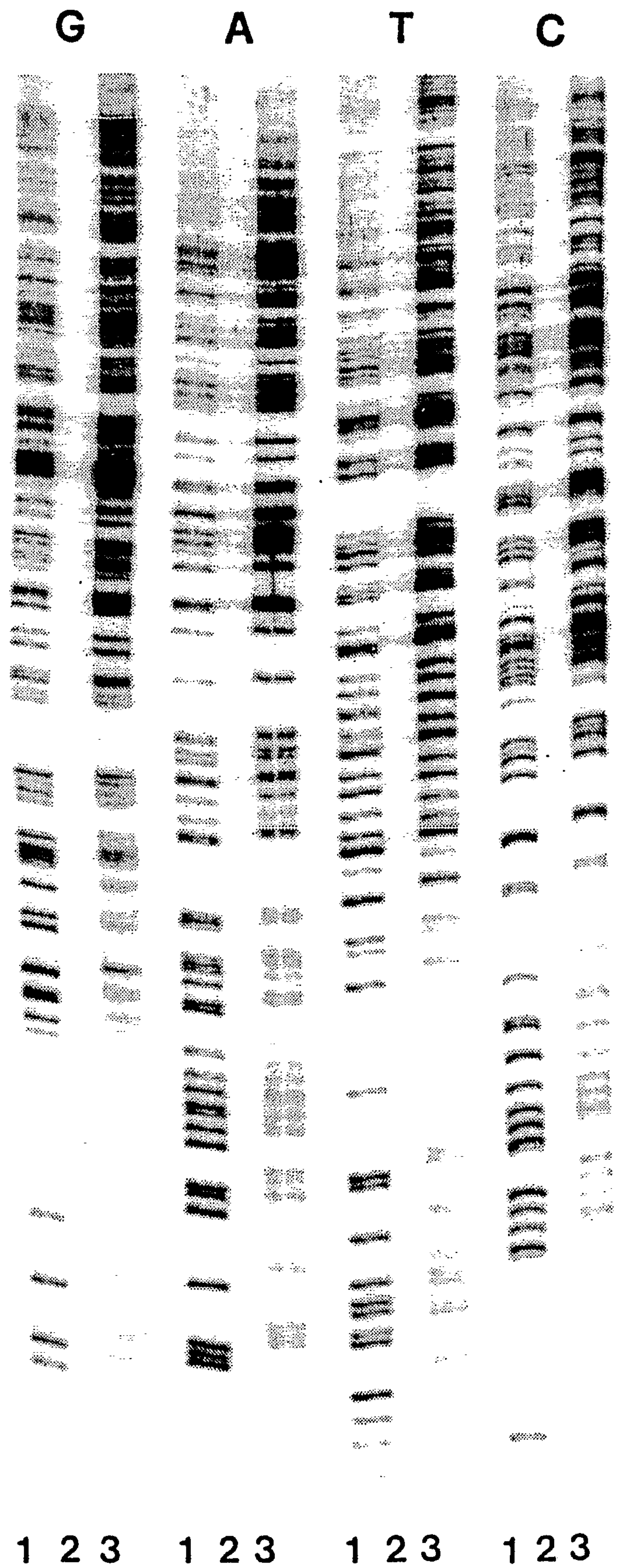

Figur 2

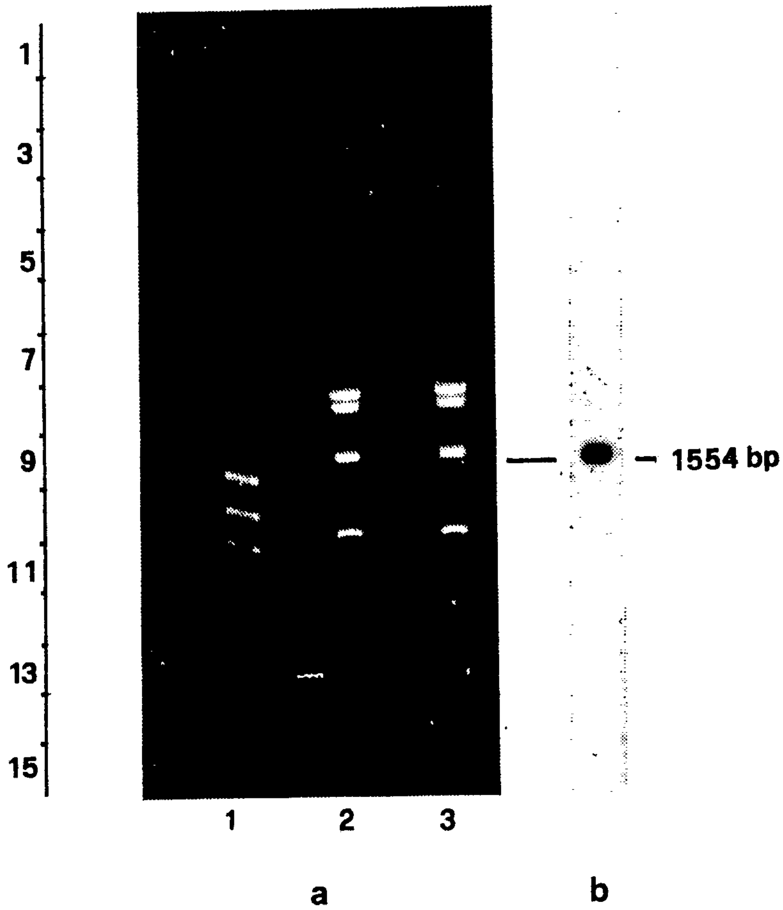


a: Auftrennung der SspI Verdauung

b: Spezifische Hybridisierung mit dem 1554 bp Fragment.